# EUROPEAN PATENT APPLICATION

(11) **EP 4 552 586 A1**
(43) Date of publication of application: **14.05.2025**
(21) Application number: 23209125.6
(22) Date of filing: 10.11.2023
(51) Int. Cl.: A61B 17/04

(54) **MEDICAL FELTING SYSTEM AND POWER UNIT**

(71) Applicant: ZuriMED Technologies AG, 8008 Zürich (CH)
(72) Inventor: BRUN, Sascha, 8008 Zürich (CH); SPOTHELFER, Dominic, 8008 Zürich (CH); LI, Xiang, 8008 Zürich (CH); BACHMANN, Elias, 8008 Zürich (CH)
(74) Representative: Vossius & Partner Patentanwälte Rechtsanwälte mbB

(57) **Abstract**

The disclosure relates to a medical felting system (1). The medical felting system comprises a felting device (200) and a power unit (100). The felting device comprises at least one felting needle (203) that is configured to move back and forth between two end positions. The power unit comprises a battery (155) that stores electrical energy, an electrical motor (165), and a controller (164). The electrical motor is configured to actuate the at least one felting needle. The electrical motor is electrically connected to the battery. The controller is configured to control the electrical motor and to actuate the motor to drive the felting needle. The felting device and the power unit are separable from one another. The felting system comprises a mechanical coupling (300) that connects the power unit operatively and releasably to the felting device. The mechanical coupling transfers rotational motion from the motor to the at least one felting needle.

## Description

The present invention relates to a medical felting system and a power unit.

Recently, the present applicant has developed a new affixation technique for soft tissues such as skin, muscles, tendons, etc. In this technique, a nonwoven material is felted to soft tissue. The nonwoven material comprises a multitude of fibers in the form of a matting or felted patch. Some of the fibers of the felted patch are pushed or pulled through the implant into the soft tissue producing a connection between the felt material and the soft tissue. To push and pull the fibers of the felted patch through the implant into the soft tissue, a felting system is provided. One example felting system is disclosed in WO 2020/227838 A1.

Any tools and devices that are used in a clinical environment and in particular during operations must be sterile. Felting systems such as shown in WO 2020/227838 A1 may be used invasively or non-invasively in operations. Thus, these devices must be sterile prior to use. Generally, a new sterile felting system may be used each time. While this approach may be convenient, it may generate excessive waste and it may be expensive to replace the entire tool after a single use. Otherwise, a felting system may be sterilized after an operation. However, sterilization binds hospital resources. Further, sterilization often includes heating portions of the device to, e.g., 121 to 134 °C and applying steam to the device. This process is aggressive and may damage parts of a felting system. An additional problem is that complex structures are difficult to sterilize, leading to long sterilization times.

Further, the recently introduced felting technique is particularly useful in arthroscopic applications such as a rotator cuff tendon repair. In arthroscopic applications, freedom of movement of an operator is particularly important. However, previously disclosed felting systems required a wire connection for the power supply, making the devices inflexible.

The present disclosure is intended to address one or more of the above problems. In particular, it is an object of the present disclosure to provide a reusable felting system that may be safe to use.

A first aspect of the present disclosure relates to a medical felting system. The medical felting system comprises a felting device and a power unit. The felting device comprises at least one felting needle that is configured to move back and forth between two end positions. The power unit comprises a battery that stores electrical energy, an electrical motor, and a controller. The electrical motor is configured to actuate the at least one felting needle. The electrical motor is electrically connected to the battery. The controller is configured to control the electrical motor and to actuate the motor to drive the felting needle. Driving the felting needle may be understood as moving the felting needle back and forth between the two end positions. The felting device and the power unit are separable from one another. The felting system comprises a mechanical coupling that connects the power unit operatively and releasably to the felting device. The mechanical coupling transfers rotational motion from the motor to the at least one felting needle.

A felting device as mentioned herein may be understood as a portion of the device that includes the at least one felting needle. The felting device may additionally include one or more of: an outer housing, an inner frame, a guiding bore, a biasing element, a spring, a yoke, a sliding pin, and a buckling element. The outer housing may enclose the felting device.

Preferably, the felting device is for single-use and thrown away after use. The felting device is particularly contaminated during use and may include parts that are difficult to sterilize. For example, a removal of the needle alone introduces health hazards for an operator as the needle may have been in contact with bodily fluids such as blood that can contain pathogens of infectious diseases. Discarding the needle may further require a specialized needle container, e.g. a cannula disposal container. In addition, inserting a new needle introduces the risk of injuries, especially with the sharp tip. Providing a separable felting device avoids these risks. An example felting device is disclosed in CH000247/12023.

The battery may be rechargeable and/or replaceable and may include one or more electrochemical cells. Any cell chemistry may be used though lithium-ion batteries are preferred. The battery may store sufficient energy to actuate the needle for the duration of an operation, e.g. at least 1, 2, 5, 10, 15, 30, or 60 minutes. The battery may include a battery pack including a plurality of identical batteries or cells. The battery enables a free movement of the system without requiring cables for a power supply. Thus, a more mobile and easier to use felting system is provided. Further, the same power unit may be re-used after an operation and can be flexibly exchanged.

The electrical motor may be configured to operate at 1,000 to 5,000 RPM, preferably at 2,000 to 3,000 RPM. The electrical motor may generate a torque of at least 10 mNm, 20 mNm, 25 mMn, or 28 mNm. The electrical motor may be a DC or AC motor. DC motors are preferred as they do not require power electronics when energy is supplied from batteries may provide high rotation frequencies. In one particular example the motor is a DCX22L GB KL 36V Maxon Motor.

The controller may include control electronics which may comprise a microcontroller.

Preferably, the felting device comprises only one felting needle. When a felting needle is mentioned herein in the following, this may mean only one felting needle or at least one felting needle, i.e., one, two, three, or more felting needles.

The two end positions may be a distal end position and a proximal end position. The felting needle may be fully retracted within a housing of the felting device in the proximal end position. In the retracted position the needle tip is protected, e.g. by the outer housing, and injuries with the needle tip can be prevented. Preferably, the felting device additionally comprises a spring configured to bias the felting needle to the proximal end position, i.e. the retracted position.

Preferably, the mechanical coupling comprises a slit for receiving an input shaft of the felting device. Correspondingly, the input shaft may comprise a plate-shaped end portion that may engage the slit. The slit allows for an easy attachment.

Preferably, the electrical motor comprises a motor output shaft and the mechanical coupling comprises a coupling connector. The coupling connector may receive a motor output shaft and includes the slit. The coupling connector may receive the motor output shaft directly or indirectly, e.g., there may be a motor adapter between the coupling connector and the motor output shaft. The coupling connector allows releasable connection between the power unit and the felting device.

Preferably, the mechanical coupling comprises first slanted edges to rotate the mechanical coupling. The mechanical coupling engages an input shaft of the felting device. The slanted edges may be provided on one or more side surfaces that define the slit. The slanted edges may define angled, in particular rounded surfaces that may define side surfaces of the slit. The side surfaces may be configured to rotate the coupling connector to align with the current position of the felting needle. Thus, the felting needle may remain in a retracted position when attaching the power unit to the felting device. No movement of the felting needle may be caused by the attachment. Further, a user may directly connect the power unit to the felting device without having to check the current position of the input shaft, the coupling connector, the motor or the power unit.

Preferably, the mechanical coupling comprises second slanted edges to rotate the mechanical coupling when the mechanical coupling engages a motor shaft. The second slanted edges may be provided additionally or alternatively to the first slanted edges and may have the same or a different shape.

Preferably, the mechanical coupling is free to rotate when the electrical motor is not actuated. For example, the motor may be free to rotate.

Preferably, the device comprises a manual trigger. The manual trigger may be configured to move from a first position to a second position. A movement of the manual trigger to the second position causes the controller to actuate the electrical motor. The manual trigger may be part of the felting device or the power unit. However, preferably the manual trigger is part of the power unit. Thereby, all electrical parts can be kept in the power unit.

Preferably, the manual trigger is biased towards the first position. Example biasing elements are springs, but any elastically deformable material may also be suitable.

Preferably, the manual trigger comprises a locking mechanism. The locking mechanism may be a mechanical lock or electrical lock. The locking mechanism may prevent actuation of the manual trigger when the locking mechanism is activated. For example, the locking mechanism may prevent a movement of the manual trigger. In other embodiments, electrical connections of the manual trigger are deactivated or disabled or blocked. The locking mechanism prevents inadvertent actuation of the felting needle.

Preferably, the controller is configured to store or stores a zero position of the motor upon engagement of a mechanical coupling with the felting device. The mechanical controller may include a memory, preferably a nonvolatile memory, to store the zero position. The zero position may be a zero position of the motor. In some embodiments the zero position may be a zero position of the motor and the coupling. The motor may comprise an encoder. The encoder may detect a current position, e.g. an angle position, of the motor. In the zero position of the motor, the felting needle may be in the retracted position.

When the motor is supplied with power, e.g. when power is supplied to the coils of the motor, it may apply a stopping torque. Thus, the controller may not supply the motor with power when the felting device is separated from the power unit or at least during attachment of the felting device and the power unit. Then, the motor may rotate freely when the power unit is connected to the felting device. The zero position may be a position of the motor after attachment of the felting device and the power unit to each other.

The felting device may comprise a biasing element, e.g. a spring, that biases the needle towards the retracted position. The same or a different biasing element may rotate the motor into the zero position upon engagement. Upon engagement, the motor shaft may align with the retracted position. For example, the motor may rotate into a motor position that corresponds to the retracted needle position.

Preferably, the controller is configured to store the zero position, e.g., upon powering the motor. The motor may alternatively or additionally store the zero position upon the power unit being switched on and/or upon detecting an attachment between the power unit and the felting device.

Preferably, the controller is configured to return the motor to the zero position after the motor was actuated. This ensures that the needle is held in the retracted position after use of the device.

Preferably, the controller is configured to control the motor to actuate the at least one needle at a constant speed during operation. The motor may maintain a constant speed. Constant speed may mean a constant rotation speed, e.g., a constant number of rounds per minute (rpm). For example, the controller may increase a motor torque, e.g. by applying a higher voltage, when the speed of the motor slows down. In one example, the controller may include a PI or PID controller that controls the motor speed to a set motor speed.

During operation, the needle may move through soft tissue and the non-woven material, and a corresponding friction may slow the movement speed of the needle down and/or may cause a nonregular movement of the needle. For example, the movement speed of the needle may depend on the felted implant and on the felted soft tissue. A slower or even irregular needle movement may reduce the attachment that is achieved when felting for a set amount of time. In order to avoid an uneven attachment of an implant, the movement speed is kept constant using the controller, even in adverse felting environments.

Preferably, the felting device comprises a felting device housing. The power unit comprises a power unit housing. The power unit housing encompasses the battery, the electrical motor and the controller. The power unit housing protects the electrical parts.

Preferably, the motor and/or the battery are removable from the power unit housing. In one example, the power unit housing includes a first end that is attached to the felting device and a second opposing end. The second opposing end may be a bottom end. Preferably, the motor and/or the battery may be removed from the bottom end.

Preferably, the power unit housing encompasses an inner housing that encompasses the motor and/or the battery. Thereby, the motor and/or the battery can be easily removed from the power unit housing. This may simplify charging, cleaning, and sterilizing the power unit.

Preferably, the inner housing or the outer motor housing comprises spring-loaded electrical connectors electrically connecting the motor and/or battery and the controller. This ensures a reliable electrical connection between the components held in the inner housing, e.g. motor and/or battery, and the remainder of the power unit.

Preferably, the inner housing comprises a first inner housing portion housing the battery and a second inner housing portion housing the motor. The first inner housing portion and the second inner housing portion may be releasably connectable to each other.

Preferably, the electrical motor is configured to drive a motion of the felting needle between the two end positions.

A further aspect of the present invention relates to a power unit according to one of the previous claims. The power unit may be sterilizable. Sterilization may involve applying steam at temperatures of 121 to 134°C for extended periods of time, e.g., 30 to 60 minutes. The power unit being sterilizable may mean that the power unit can withstand this procedure. Further, as e.g. outlined above, certain elements of the power unit such as the battery and/or the motor may be removable such that only parts, e.g. only the power unit housing, may need sterilization. In one example all electrical components are removable and only the remaining parts are sterilized, in particular only the mechanical parts (e.g., power unit housing, the trigger, etc.) may be sterilizable.

Further features and advantages of the invention shall be described by means of a detailed description of embodiments with reference to the figures. The figures illustrate example embodiments that do not limit the scope of the present disclosure.
Figure 1 shows a side view of a felting system.
Figure 2 shows a side view of an exploded view of a felting system, wherein an outer housing of a power unit is removed.
Figure 3 shows a cross-section of the felting system shown in figure 1 in assembled form.
Figure 4 shows a side view and a perspective view of a coupling connector.
Figure 5 shows a perspective view of a power unit.

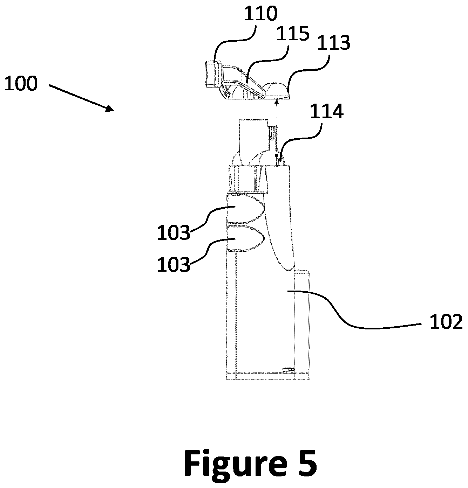

Figure 5 shows different embodiments of a trigger.

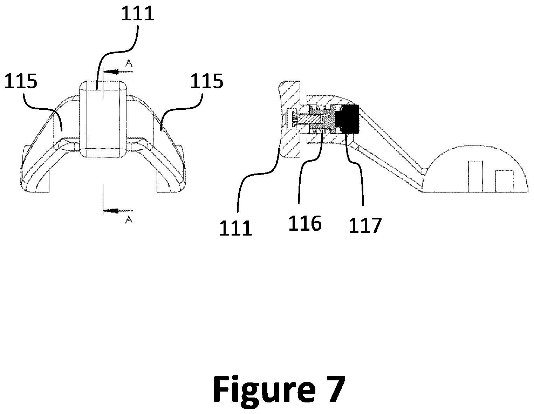

Figure 7 shows a further example trigger and a cross-section of the trigger.

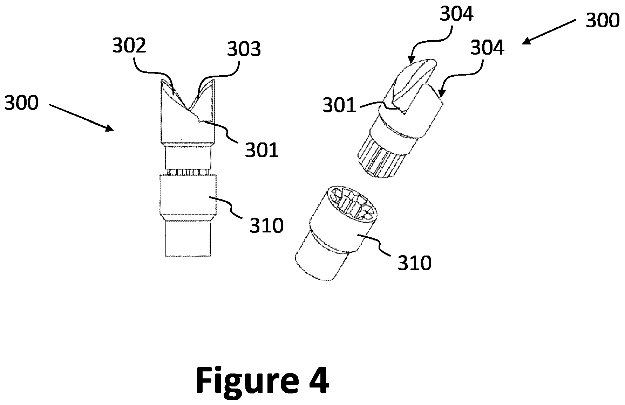

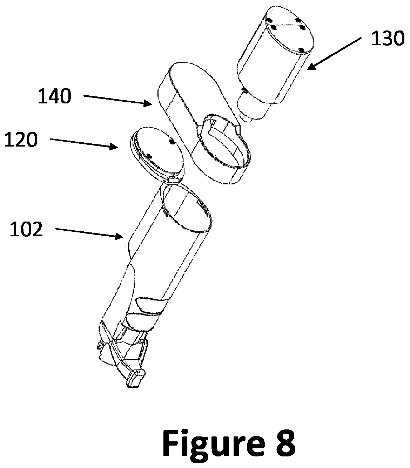

Figure 8 shows an exploded view of the power unit of figure 4 and an aseptic transfer shield.

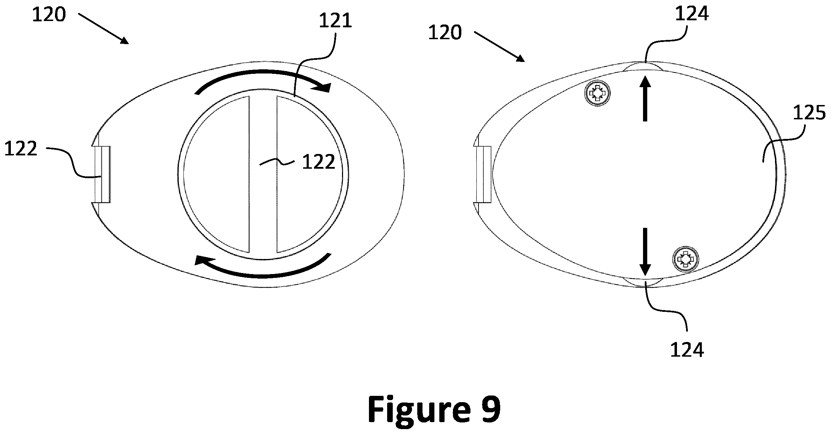

Figure 9 shows a latch of the power unit of figure 4 in a closed position.

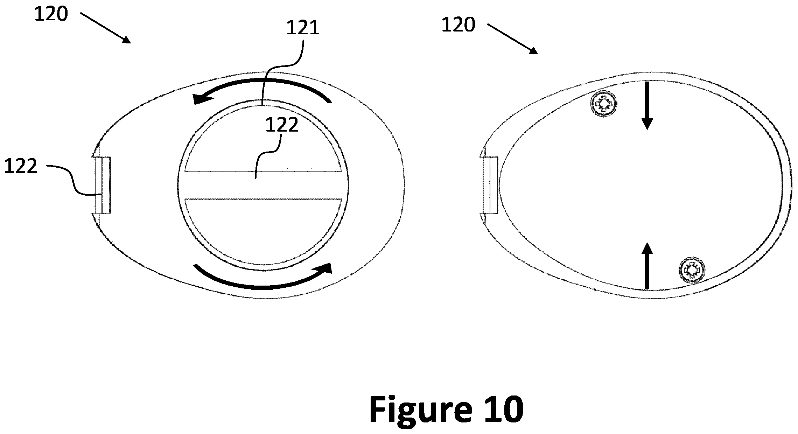

Figure 10 shows the latch of the power unit of figure 4 in an open position.

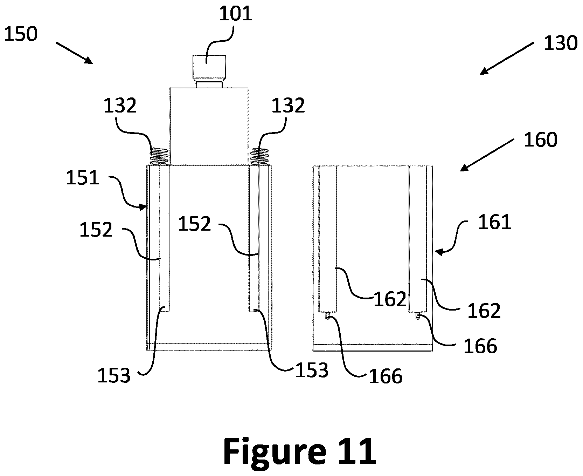

Figure 11 shows a side view of a battery unit and motor unit when separated from each other.

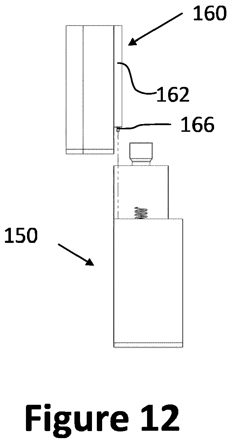

Figure 12 shows a connection between the battery unit and the motor unit.

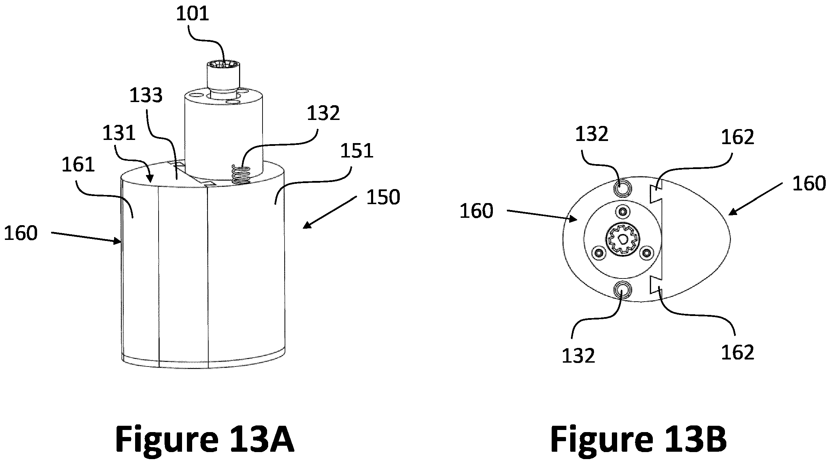

Figure 13A shows a battery unit and a motor unit when connected to each other in a perspective view.
Figure 13B shows a top view of the battery and motor unit of figure 12A.

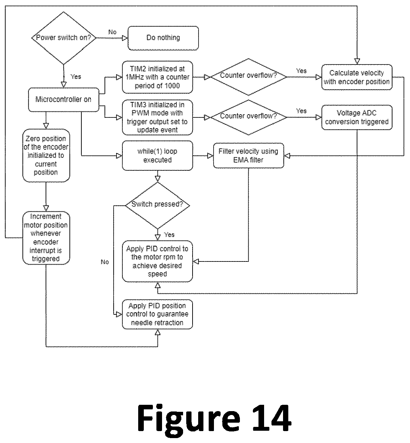

Figure 14 shows a control logic of a controller of the power unit.

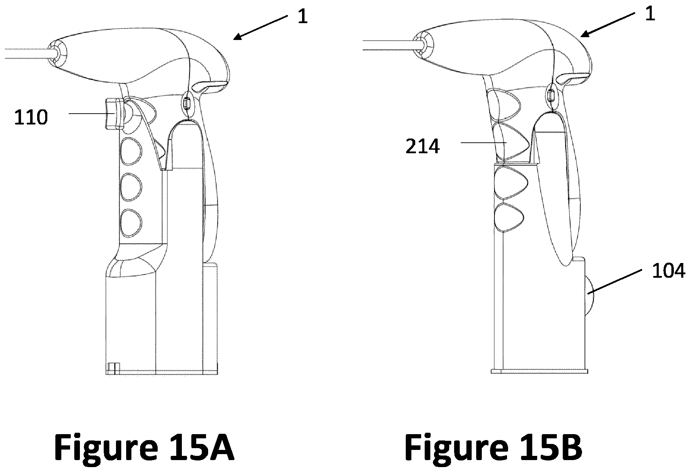

Figures 15A and 15B show alternative felting systems.

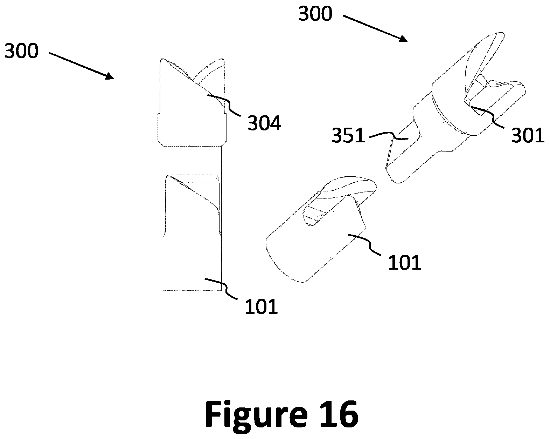

Figure 16 shows a side view and a perspective view of a second embodiment of a coupling connector.

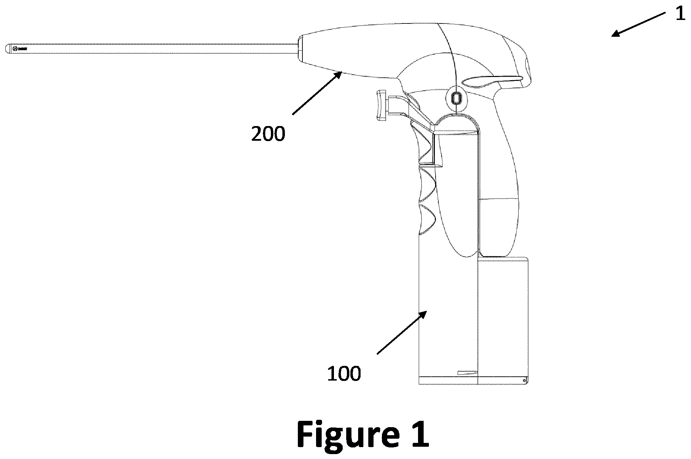

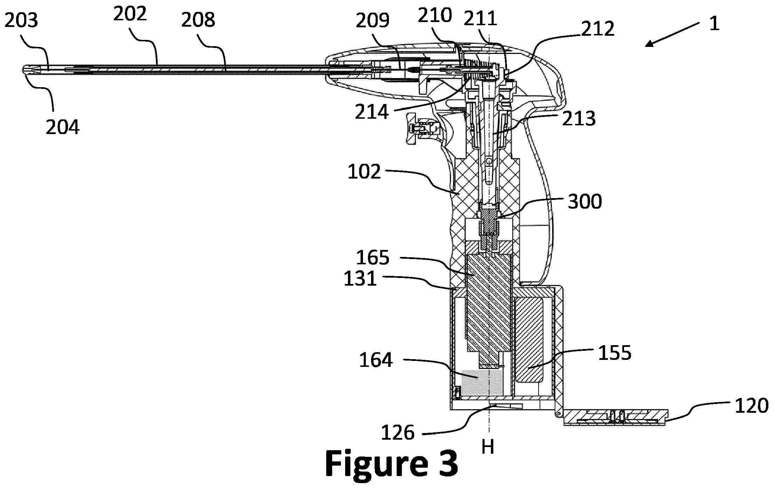

Figures 1 to 3 show a felting system 1 that comprises a power unit 100 and a felting device 200. The felting device 200 includes an outer housing 201 and a tubular member 202 extending distally from the outer housing. The directions proximal and distal as used herein are defined from the perspective of an operator of the felting system. Proximal may generally define a direction towards the operator and distal may generally define a direction away from the operator holding the felting system in the assembled form. The tubular member 202 holds a felting needle 203 (see figure 3). During operation, the felting needle 203 is moved along a longitudinal direction of the tubular member and expelled and retracted from a distal end 204 of the tubular member 202. The outer housing 201 may include a proximal part 205 and a distal part 206. The outer housing may be made of a rigid plastic material. A driving mechanism for the felting needle 203 is arranged within the outer housing 201. As can be seen from figure 3, the driving mechanism may include a guide rod 208 that holds the felting needle at its distal end. The guide rod 208 is connected to a buckling element 209, which then connects to a piston rod 210 that terminates in scotch yoke 211. Guide rod 208, buckling element 209, piston rod 210 and scotch yoke 211 are arranged along the longitudinal direction of the felting needle 203, connected to each other and move together along the longitudinal direction of the felting needle 203. Sliding pin 212 extends in parallel to a handle axis H and is arranged inside the scotch yoke 211. The pin 212 is connected to a top end of a felting device input shaft 213. During actuation the felting device input shaft rotates around its axis which also rotates the pin 212 around the axis of the input shaft 213. This actuates the scotch yoke 211 and causes a back-and-forth motion between two end positions of the felting needle 203. In addition, the felting device 200 includes a biasing element, e.g. a spring 214. The spring 214 pushes the piston rod 210 and thus the felting needle 203 to their proximal most position. The proximal most position may be a retracted position. Further details of the felting device are described in the application CH000247/12023.

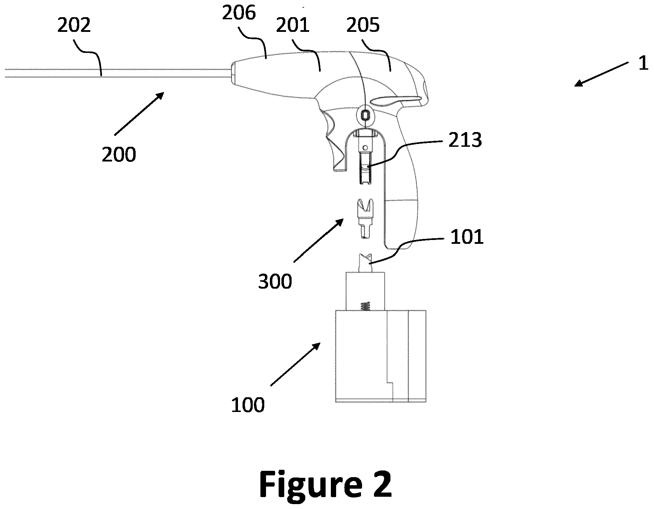

Figures 2 to 4 also show a coupling connector 300. The coupling connector 300 includes a first side, that is directed towards the felting device 200 and a second side that is directed towards the power unit 100. The first side of the coupling connector 300 includes a slit 301 (see figure 4) the slit 301 has a straight shape and is limited by side surfaces 302 and 303. The slit 301 may be similar to a slit of a flathead screw. The slit 301 may be formed by two extensions from a body of the coupling connector 300. The extensions may have a fork shape. The slit may be formed between two prongs of the fork shape. The first side is adapted to receive the felting device input shaft 213. An end portion of the felting device input shaft 213 has the shape of a flathead screwdriver. The end portion may include flat side surfaces that may extend along the direction H. The flat side surfaces may fit into the slit 301 of the coupling connector 300.

Further, as can be seen from figure 4, the first side includes first slanted edges 304. The slanted edges 304 may cut away a portion of the side surfaces 302 and/or 303 and may provide an angled guide surface with respect to longitudinal axis H or a rotation axis of the coupling connector 300. The angled guide surface may also be angled with respect to the plane perpendicular to the longitudinal axis H. Further, the angled guide surface may be flat. However, in preferred embodiments, the angled guide surface has a rounded shape to guide rotation of the felting device input shaft 213. The angled guide surface may be convex or concave. Each side of the slit 301, e.g. each prong of the fork shape, may include a slanted edge 304, though in some embodiments only one side of the slit 301 may include the slanted edge 304.

As can be seen in figures 2 and 16, the second side of the coupling connector includes a flathead shape 350, similar to the end portion of felting device input shaft 213. The second side may include two flat outer side surfaces 351 for rotational engagement. In this embodiment, the second side may have the shape of a flathead screwdriver, and/or may have the shape of an end portion of the felting device input shaft 213. The second side may include flat outer sidewalls extending in parallel to the longitudinal axis of the coupling connector. In this embodiment, a motor output shaft 101 may include the shape described and shown with respect to the first side of the coupling connector 300.

In a second embodiment, the second side of the coupling connector 300 may include a standard connection interface, e.g. a hex key (or any other amount of edges) or any other known screw drive mechanism. In the embodiment shown in figure 4, the second side may have a constant cross-section and may fit in multiple rotational positions for easy assembly. The shown second side of figure 4 has a shape similar to an external polydrive, though more than six flat teeth are provided. The shown embodiment includes eight cam shaped teeth which allows the transmission of high torques. The cam shaped teeth are arranged directly next to each other and extend in parallel to each other.

The coupling connector may include a motor adapter 310. The motor adapter 310 includes a first side facing the coupling connector and a second side for attachment to the motor. Some motors may have a motor shaft with a D - shape (sometimes described as type D). The second side may include a corresponding inverse D shape to fit onto the motor output shaft 101. The first side may fit into the second side of the coupling connector and may be correspondingly shaped.

Figure 3 shows the felting system including the power unit 100 and the felting device 200 in an assembled state. A motor 165 actuates the motor output shaft 101. The motor output shaft 101 rotates adapter 310 and coupling connector 300. These in turn actuate the felting device input shaft 213 and thus the felting needle 203.

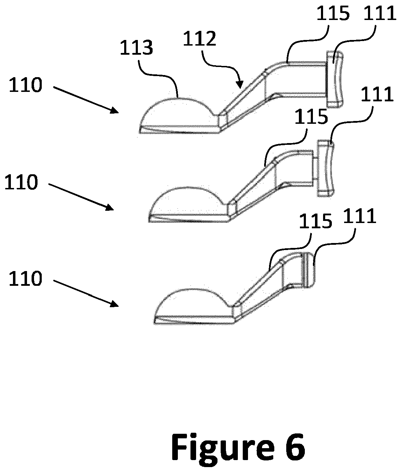

Figure 5 shows an embodiment of the power unit 100 in isolation. Generally, the power unit 100 includes a power unit housing 102, that may be made of a rigid plastic material. The power unit housing 102 may or may not use the same material as the housing 201 of the felting device. Generally, the power unit housing 102 has a cylindrical shape. A front side of the power unit housing 102 includes concave indentations 103 for gripping the power unit 100. The power unit includes a manual trigger 110 that is illustrated in more detail in figures 6 and 7. The manual trigger 110 is arranged at a front facing side and may include a button 111. Pressing the button may actuate the motor as will be described below in greater detail.

The manual trigger 110 includes a trigger housing 112. The trigger housing 112 may include a trigger base 113 that attaches to a top side of the power unit housing 102. The power unit housing 102 may include two pins 114 and the trigger base 113 may include corresponding recesses that fixate the trigger housing 112 on the power unit housing 102. Two bars 115 extend from the trigger base 113 and hold the button 111 jointly. The bars 115 may include electrical wiring that connects the button 111 with a controller held in the power unit 100. Figure 7 shows a cross-section of the trigger housing 112. As can be seen, button 111 is biased outwardly by a spring 116 which ensures that the button 111 is deactivated as a default position. Electrical switch 117 is activated upon pressing button 111 beyond a threshold against the force of the spring 116.

Figures 6, 15A, and 15B show alternative manual triggers 110. In figure 6, different sizes of button 111 and orientations of the bars 115 are shown. In figure 15A, the manual trigger is directly mounted on a front side, i.e. a proximal side, of the power unit housing 102. In figure 15B, the manual trigger 110 is arranged beneath an indentation 214 of the felting device. The trigger is actuated by compressing the housing of the felting device. Further, the power unit 100 may comprise an on/off switch 104.

Figure 8 shows an exploded view of the components held within power unit housing 102. The power unit housing 102 includes a bottom latch 120. Opening the bottom latch 120 enables a release of a motor and battery unit 130. The motor and battery unit 130 can be slid in and out of the outer housing through the opening provided at a bottom of the power unit housing 102. In some embodiments, additionally, an aseptic transfer shield 140 may be provided. The aseptic transfer shield 140 may allow insertion of the motor and battery unit 130 without having to touch power unit 100. The latch 120 may be opened, the aseptic transfer shield 140 may be placed onto the bottom end of the housing 102 and then the motor and battery unit 130 may be removed or inserted without contacting the inside surface of the latch 120. Thereby, a risk of contamination of the outside components of the power unit 100 or a risk of contamination of a person handling the power unit 100 may be reduced.

In one example, the power unit housing 102 may be sterile on the outer side. A fully charged motor and battery unit 130 may be nonsterile. An operator (e.g. a nurse) may insert the motor and battery unit 130 through the aseptic transfer shield 140 into the sterile power unit housing 102. The additional use of the aseptic transfer shield 140 prevents any contact of the nonsterile motor and battery unit 130 with the sterile outer surface of the power unit housing 102. Thus, even after exchanging the motor and battery unit 130, the power unit housing 102 of the power unit 100 may not need a re-sterilization. The aseptic transfer shield 140 may be provided in a set with the power unit and or with the felting system described herein.

Removal of the motor and battery unit 130 from the bottom of the power unit 100 may be preferred as this may allow for a battery/motor replacement without having to remove the felting device from the power unit.

Figures 9 and 10 show the latch 120 in more detail. Figures 9 and 10 each show a bottom view of the latch 120 on the left side and a top view (i.e. from inside of the body 102) of the latch 120 on the right side. The latch 120 may have a rounded shape. In the shown example, the latch 120 has an oval shape though other shapes such as an egg shape may also be suitable. The latch 120 comprises a round, i.e. circular, handle 121 which may include a straight bar 122 extending from edge to edge through a center of the circular handle 121. The circular handle 121 may be rotated to lock and an unlock the latch 120. Rotation of the circular handle 121 causes locking ends 124 to rotate as well. As can be seen from the comparison of figure 9 and figure 10, clockwise rotation (see figure 9 left side) causes the locking ends 124 to move behind interior bottom plate 125 of the latch 120 (see figure 10, right side). The locking ends 124 may interlock with noses 126 (see figure 3). Noses 126 may be provided on an interior wall of the power unit housing 102, in particular in a bottom part of the cylindrical portion of power unit housing 102. The locking ends 124 may be provided as ends of a flat bar. In addition, connection means, i.e. screws, may connect the flat bar with the locking ends and the handle 121 such that they rotate jointly. The rotation of the handle may be limited, e.g. using a stopper.

Further, the latch comprises a joint 122 with which the latch 120 is rotatably attached to the power unit housing 102. After the handle 121 is rotated into the open position, latch 120 may rotate around joint 122 and the motor and battery unit 130 may be removed.

Figures 11 to 13B show the battery and motor unit 130. The battery and motor unit 130 includes an outer casing 131 (see figure 13A) that can be separated into a motor unit 150 and a battery unit 160. Each of these units includes a respective casing 151 and 161 housing respective motor 165 and battery 155 (see figure 3). The casing 151 of the motor unit 150 generally extends along a longitudinal axis that extends in the same direction as the output motor shaft to which the coupling connector 300 is attached. The same applies for the casing 161 of the battery unit 160. The motor casing 151 includes longitudinal extending grooves 152 and the battery casing includes correspondingly shaped rails 162. As illustrated in figure 12, the rails 162 may be slid into the grooves 152 to attach and release the motor unit 150 and the battery unit 160 from each other. This enables the battery unit 160 to be charged separately from the motor unit 150. Also, the battery unit 160 may be charged while connected to the motor unit 150. The battery unit 160 may include electrical charging connectors 166. Alternatively or additionally, the battery unit 160 may comprise wireless charging circuitry. As can be seen from figure 11, the rails 162 and corresponding grooves 152 extend from a top surface 131 along the longitudinal axis without reaching a bottom end of the motor unit casing 151 and the battery unit casing 161. An end of the grooves 152, preferably a bottom end, may include one or more spring-loaded electrical connectors (not shown). Alternatively, an end of the rails 162, preferably a bottom end may include one or more spring-loaded electrical connectors. This provides for a reliable electric connection between the battery unit 160 and the motor unit 150.

Generally, the battery and motor unit 130 has a cylindrical shape with an oval cross-section, e.g. a similar shape as the housing 102. Parts of the motor unit 150 may extend from the cylindrical shape. The oval-shaped prevents insertion of the battery and motor unit 130 in an undesirable orientation. The battery and motor unit 130 may include a top surface 133 on which upwardly facing electrical spring-loaded connectors 132 are provided. These connectors connect to corresponding electrical connectors inside outer housing, for example to the manual trigger described above. Though spring-loaded connectors are preferred, electrical conductors without a spring loading may also be used.

Control electronics 164 in the motor housing 160 (see figure 3) may detect a connection between the upwardly facing electrical spring-loaded connectors 132 and the manual trigger 110.

Assembly of the felting system 1 may include the following steps. First, the battery and motor unit 130 may be inserted into the power unit housing 102 of the power unit 100. Then, latch 120 is closed. Closing of the latch 120 presses the battery and motor unit upwardly along the longitudinal axis of power unit housing 102 and against spring-loaded connectors 132. In this position, the latch 120 is locked. The coupling connector 300 and adapter 310 may be fixedly attached to the motor output shaft 101 or may be manually slid onto the motor output shaft 101 after insertion of the battery and motor unit 130. After assembly of the power unit 100, the power unit 100 and the felting device 200 are connected to each other. When connecting the power unit 100 and the felting device 200, the felting device input shaft 213 is moved into the coupling connector 300. During this assembly, the motor 165 may not be supplied with power from the battery 155. In one example, the control electronics 164 may be switched off. In another example, the control electronics 164 may switch a power supply off during assembly. This allows the motor 165 to rotate freely. The spring 214 biases the rotational position of the felting device input shaft towards a position in which the needle 203 is in a retracted position. During attachment, the slanted edges of the coupling element cause the motor to rotate into the position of the felting device input shaft 213.

The battery and motor unit 130 also includes control electronics 164, e.g. a microcontroller. The control electronics may check if the trigger and the battery are accurately connected, e.g. using spring-loaded connectors 132.

Figure 14 shows a control logic of the control electronics. The control logic may further verify whether the felting device 200 and the power unit 100 are accurately connected, e.g. using further electrical connectors. As long as the control electronics 164 are switched off, the motor is not energized. Upon switching the control electronics 164 on (e.g. using a switch or trigger), the current position of the motor is read out by the control electronics 164. The motor 165 may comprise an encoder that may transmit a current position of the motor to the control electronics 164. If the power unit 100 and the felting device 200 have been accurately connected, this current position corresponds to the retracted position of the felting needle 203. The current position is then stored as a zero position in the control electronics 164.

The control electronics 164 monitors if the trigger is pressed ("while (1) loop executed"). While the trigger is not pressed, the control electronics maintains the motor in the zero position and thus the felting needle 203 in the retracted position. In one example, the control electronics 164 may include a PID controller for controlling the position of the motor. Whenever the encoder detects a deviation from the zero position, e.g. when the encoder interrupt is triggered, the control electronics 164 signals the motor to return to the zero position. The motor may rotate in both directions, e.g. clock-wise and counterclockwise. Preferably, the control electronics selects the least amount of rotation to return to the zero position. This avoids that the needle 203 is pushed in a distal direction and reduces a risk of injury.

If the trigger is pressed, the motor is actuated at a predetermined rotational speed, e.g. at 2,500 RPM. Whenever an encode interrupt is triggered, the detected motor position is incremented. A timer ("TIM2") causes the microcontroller to calculate the current motor velocity (angular velocity) in set time intervals. In the shown example of figure 14, this occurs every 1 ms ("1 MHz with a counter period of 1,000"). The measured velocity is filtered with an exponential moving average filter. Then the measured and filtered velocity is fed into the PID controller. The PID controller keeps the rotational speed constant using the feedback from the encoder. The actuation of the motor is controlled using PWM (Pulse Width Modulation). A timer ("TIM3") is initialized for the PWM. The pulse width modulation may result in an average voltage that determines the applied torque and/or rotational velocity. If the motor runs too fast, the average voltage and thus applied torque and/or rotational velocity may be decreased using PWM. If the motor runs too slow, the average voltage and thus applied torque and/or rotational velocity may be increased.

Further aspects of the disclosure are described in the following:
1. A medical felting system comprising:
   a. a felting device comprising at least one felting needle that is configured to move back and forth between two end positions; and
   b. a power unit comprising:
      i. a battery that stores electrical energy,
      ii. an electrical motor for actuating the felting device, the electrical motor being electrically connected to the battery,
      iii. a controller for controlling the electrical motor, the controller being configured to actuate the motor to drive the felting device, and
   c. wherein the felting device and the power unit are separable from each other and wherein the felting system comprises a mechanical coupling that connects the power unit operatively and releasably to the felting device, wherein the mechanical coupling is configured to transfer a rotational motion from the motor to the at least one felting needle.
2. Medical felting system according to aspect 1, wherein the mechanical coupling comprises a slit for receiving an input shaft of the felting device.
3. Medical felting system according to aspect 2, wherein the electrical motor comprises a motor output shaft and wherein the mechanical coupling comprises a coupling connector, wherein the coupling connector receives the motor output shaft and includes the slit.
4. Medical felting system according to one of the previous aspects, wherein the mechanical coupling comprises first slanted edges to rotate the mechanical coupling when the mechanical coupling engages an input shaft of the felting device.
5. Medical felting system according to one of the previous aspects, wherein the mechanical coupling comprises second slanted edges to rotate the mechanical coupling when the mechanical coupling engages a motor shaft.
6. Medical felting system according to one of the previous aspects, wherein the mechanical coupling is free to rotate when the electrical motor is not actuated.
7. Medical felting system according to one of the previous aspects comprising a manual trigger, the manual trigger being configured to move from a first position to a second position, wherein a movement of the manual trigger to the second position causes the controller to actuate the electrical motor.
8. Medical felting system according to aspect 7, wherein the manual trigger is biased towards the first position.
9. Medical felting system according to aspect 7 or 8, wherein the manual trigger comprises a locking mechanism, in particular a mechanical lock or an electrical lock, that prevents actuation of the manual trigger when the lock is activated.
10. Medical felting system according to one of the previous aspects, wherein the controller is configured to store a zero position, preferably upon engagement of the mechanical coupling with the felting device.
11. Medical felting system according to one of the previous aspects, wherein the controller is configured to store the zero position upon powering the motor.
12. Medical felting system according to aspect 10 or 11, wherein the controller is configured to return the motor to the zero position after the motor was actuated.
13. Medical felting system according to one of the previous aspects, wherein the controller is configured to control the motor to actuate the at least needle at a constant speed during operation.
14. Medical felting system according to one of the previous aspects, comprising an outer housing, wherein the outer housing encompasses the battery, the electrical motor, and the controller.
15. Medical felting system according to aspect 14, wherein the motor and/or the battery are removable from the outer housing, preferably at a bottom end opposing the mechanical coupling.
16. Medical felting system according to aspect 14 or 15, wherein the motor and/or the battery include an inner housing that houses the motor and/or the battery, and wherein the inner housing is removable from the outer housing.
17. Medical felting system according to aspect 16, wherein the inner housing comprises spring loaded electrical connectors.
18. Medical felting system according to aspect 16 or 17, wherein the inner housing comprises a first inner housing portion housing the battery and a second inner housing portion housing the motor and wherein the first inner housing portion and the second inner housing portion are releasably connectable to each other.
19. Medical felting system according to one of the previous aspects, wherein the electrical motor is configured to drive a motion of the felting needle between the two end positions.
20. Medical felting system according to one of the previous aspects, wherein the felting device additionally comprises a spring configured to bias the felting needle to a retracted position.
21. A power unit according to one of the previous aspects.
22. Power unit according to aspect 21, wherein the power unit is sterilizable.

## Claims

1. A medical felting system comprising:
a. a felting device (200) comprising at least one felting needle (203) that is configured to move back and forth between two end positions; and
b. a power unit (100) comprising:
i. a battery (155) that stores electrical energy,
ii. an electrical motor (165) for actuating the felting device (200), the electrical motor (165) being electrically connected to the battery (155),
iii. a controller (164) for controlling the electrical motor (165), the controller (164) being configured to actuate the motor (165) to drive the felting device (200), and
c. wherein the felting device (200) and the power unit (100) are separable from each other and wherein the felting system comprises a mechanical coupling that connects the power unit (100) operatively and releasably to the felting device (200), wherein the mechanical coupling is configured to transfer a rotational motion from the electrical motor (165) to the at least one felting needle (203).

2. Medical felting system according to claim 1, wherein the mechanical coupling comprises a slit (301) for receiving an input shaft (213) of the felting device (200).

3. Medical felting system according to claim 2, wherein the electrical motor (165) comprises a motor output shaft (101) and wherein the mechanical coupling comprises a coupling connector (300), wherein the coupling connector (300) receives the motor output shaft (101) and includes the slit (301).

4. Medical felting system according to one of the previous claims, wherein the mechanical coupling comprises first slanted edges to rotate the mechanical coupling when the mechanical coupling engages an input shaft (213) of the felting device (200).

5. Medical felting system according to one of the previous claims comprising a manual trigger (110), the manual trigger (110) being configured to move from a first position to a second position, wherein a movement of the manual trigger (110) to the second position causes the controller (164) to actuate the electrical motor (165).

6. Medical felting system according to claim 5, wherein the manual trigger (110) comprises a locking mechanism, in particular a mechanical lock or an electrical lock, that prevents actuation of the manual trigger (110) when the lock is activated.

7. Medical felting system according to one of the previous claims, wherein the controller (164) is configured to store a zero position, preferably upon engagement of the mechanical coupling with the felting device (200).

8. Medical felting system according to one of the previous claims, wherein the controller (164) is configured to store the zero position upon powering the motor (165).

9. Medical felting system according to claim 8, wherein the controller (164) is configured to return the motor (165) to the zero position after the motor (165) was actuated.

10. Medical felting system according to one of the previous claims, wherein the controller (164) is configured to control the motor (165) to actuate the at least needle at a constant speed during operation.

11. Medical felting system according to one of the previous claims, wherein the motor (165) and/or the battery (155) are removable from the outer housing (201), preferably at a bottom end opposing the mechanical coupling.

12. Medical felting system according to one of the previous claims, wherein the motor (165) and/or the battery (155) include an inner housing that houses the motor (165) and/or the battery (155), and wherein the inner housing is removable from the outer housing (201).

13. Medical felting system according to claim 12, wherein the inner housing comprises a first inner housing portion housing the battery (155) and a second inner housing portion housing the motor (165) and wherein the first inner housing portion and the second inner housing portion are releasably connectable to each other.

14. A power unit (100) according to one of the previous claims.

15. Power unit according to claim 14, wherein the power unit (100) is sterilizable.
